# EUROPEAN PATENT SPECIFICATION

(11) **EP 1 172 651 B1**
(45) Date of publication and mention of the grant of the patent: **15.07.2015**
(21) Application number: 01904529.3
(22) Date of filing: 19.02.2001
(51) Int. Cl.: G01N 33/48, G01D 7/12, G06Q 50/24

(54) **MEASURING SYSTEM**
MESSSYSTEM
SYSTEME DE MESURE

(30) Priority: 18.02.2000 JP 2000041713
(43) Date of publication of application: 16.01.2002
(73) Proprietor: Panasonic Healthcare Co., Ltd., Ehime 791-0395 (JP); ARKRAY, Inc., Kyoto-shi, Kyoto 601-8045 (JP)
(72) Inventor: AMANO, Yoshinori, Ehime 793-0035 (JP); TOKUNO, Yoshinobu, Ehime 791-2103 (JP); KATAOKA, Yoshihiro, Ehime 793-0035 (JP); SATO, Yoshiharu, Kyoto-shi, Kyoto 601-8045 (JP); KAWANAKA, Shoji, Kyoto-shi, Kyoto 601-8045 (JP)
(74) Representative: Balsters, Robert
(86) International application number: PCT/JP2001/001150
(87) International publication number: WO 2001/061340

(56) References cited:
- WO-A-97/36146
- JP-A- 9 094 231
- JP-A- 9 094 231
- JP-A- 60 108 715
- JP-A- 62 046 238
- JP-A- 62 046 238
- JP-U- 61 194 504

## Description

### TECHNICAL FIELD

The present invention relates to a measuring system which comprises a biosensor measuring device suitable for a biosensor that can accurately, speedily, and easily assay a specific component in various biological samples, and a complementary device that can be combined with the measuring device and complementarily announces a measuring procedure and a measurement result in the measuring device by voice.

### BACKGROUND ART

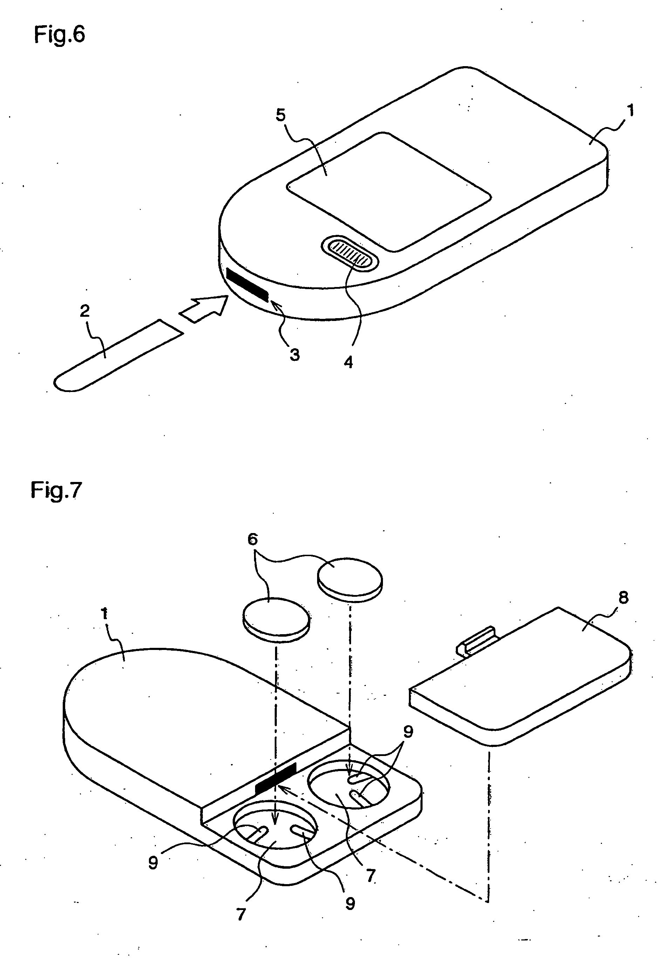

An example of a biosensor measuring device employing a biosensor will be illustrated in figures 6 and 7. A measuring device 1 is provided with a sensor insertion slot 3 in which a biosensor 2 is inserted, an operation button 4. for performing various operations, and a display means 5 for displaying various kinds of information such as a measurement result and time. A storage part 7 of a battery 6 is provided on the reverse face, and is fixed in a state where the battery 6 is pushed to a contact point 9 by a back cover 8. Inside the measuring device 1, a measuring part comprising a converting means for converting an electrical resistance value of liquid to be examined (sample) obtained by the biosensor 2 to a characteristic value (such as a blood sugar level) of the liquid to be examined, a storage means of the characteristic value, and a calculating means for performing various arithmetical operations such as an average value calculation based on a value stored in the storage means or the characteristic value obtained by measurement, a control means of the display means 5, and the like are provided by being formed on a printed board (not shown).

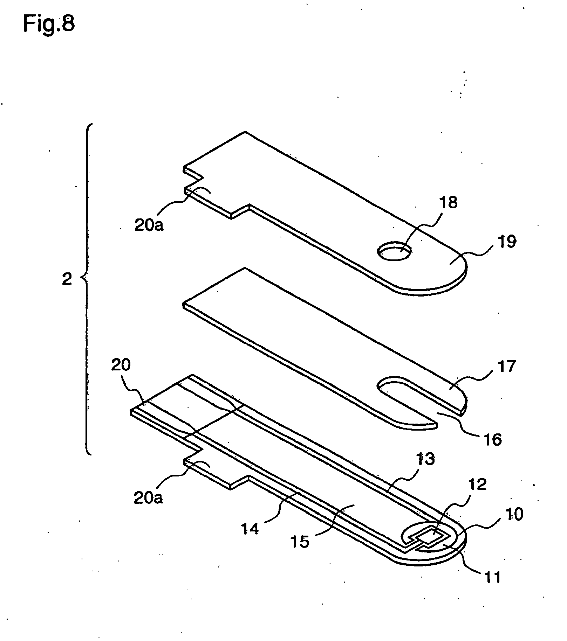

The biosensor 2 is provided with a counter electrode 11 and a measuring electrode 12, leads 13 and 14 connected thereto, and further an insulating layer 15 on the surface of a substrate 10 as shown in figure 8, as well as a reactive layer (not shown) including enzyme and a mediator (electron acceptor) is formed so as to cover the counter electrode 11 and the measuring electrode 12. The surface of the substrate 10 is sequentially covered with a spacer 17 which forms a U-shaped notch part 16 at a section corresponding to the measuring electrode 12 and a cover 19 which forms an air vent 18 connected with the notch part 16 of the spacer 17, and the substrate 10, the spacer 17, and the cover 19 are fixed with one another, thereby constructing the biosensor 2. Numeral 20 denotes an attaching end to the measuring device 1 and numeral 20a denotes a projection part provided for preventing an inverse insertion of the biosensor 2 to the measuring device 1. Therefore, in a state where the attaching end 20 of the biosensor 2 is attached to the measuring device 1, when liquid to be examined (sample) drops to the end of the biosensor 2, the liquid to be examined is led onto the counter electrode 11 and the measuring electrode 12 through the notch part 16 by capillarity, and the air on the counter electrode 11 and the measuring electrode 12 is discharged from the air vent 18.

After measurement, the characteristic value obtained by the converting means of the measuring device 1 is displayed on the display means 5.

The output of the measurement result is performed only by a display by the display means 5 in the biosensor measuring device 1 of the above-described construction. However, when a user is a weak-sighted person, for example, it is difficult to confirm the display.

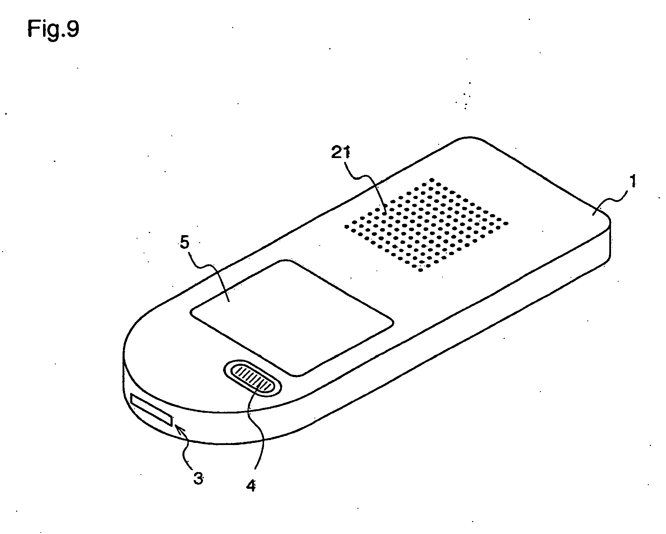

A measuring device as shown in figure 9 is invented as a countermeasure for the problem. In figure 9, the same reference numerals as those shown in figure 6 denote the same components. A voice output part 21 is provided on the surface of the measuring device 1 in addition to the display means 5, and a voice output means for announcing a characteristic value or various kinds of information by voice is provided inside the measuring device 1, besides the measuring part (both of them not shown).

However, in the above-described construction, the measuring device becomes expensive because all the parts from measurement to voice output are built in the measuring device, and, when plural users require the voice output, for example, each person is required to have the expensive device. Further, when the voice output part has a breakdown, the whole device has to be repaired and exchanged, resulting in inconvenience for a user. Similar devices are disclosed in JP 9 094231 A and JP 62 046238 A.

The present invention is made to solve the above-mentioned problems and has, for its object, to provide a measuring system which comprises a measuring device being capable of taking a substance to be measured, analyzing components of the substance to be measured, and displaying a measured value, and a complementary device having a voice output part, and can complementarily announce a measurement result and a measuring procedure in the measuring device by voice from the complementary device to a user by combining the measuring device with the complementary device.

### DISCLOSURE OF THE INVENTION

According to Claim 1 of the present invention, in a measuring system comprising a measuring device adapted to receive a biosensor for obtaining a liquid to be measured, adapted to analyze components of the liquid to be measured, and adapted to display its measurement result, and a complementary device which is detachably connected to said measuring device and which has a voice output means for generating a voice when combined with the measuring device, the complementary device is adapted to complementarily announce by voice a measuring procedure or a measurement result obtaining by the measuring device. Therefore, even a weak-sighted person or those unfamiliar with handling of the device can perform measurement accurately and be informed of a measurement result. Further, the measuring device and the complementary device can be constructed to be integrated and separated, whereby it is possible to deal with weak-sighted people and the others respectively, and thus, even when plural users require a voice function, only the measuring device which manages measurement data has to be held by each person and the complementary device can be shared, resulting in reduction of user's burden. Further, when a voice output part has a breakdown, only the complementary device has to be repaired and exchanged, and the measuring device can be used continuously, thereby enhancing convenience for a user.

According to Claim 2 of the present invention, in the measuring system as defined in Claim 1, the measuring device has a means for establishing communication of information about measurement with the complementary device. Therefore, it is possible to transfer data such as a measurement result from the measuring device to the complementary device.

According to Claim 3 of the present invention, in the measuring system as defined in Claim 1, the complementary device has a means for establishing communication of information about measurement with the measuring device. Therefore, the complementary device can receive data such as a measurement result transferred from the measuring device.

According to Claim 4 of the present invention, in the measuring system as defined in Claim 1, an operating part for operating the voice output means is arranged on the side of the complementary device. Therefore, when an operator holds the system, the operation is easily performed, thereby providing good operationality.

According to Claim 5 of the present invention, in the measuring system as defined in Claim 1, the complementary device has a function for switching a language of a voice. Therefore, it is possible to cope with a wide range of languages according to a user.

According to Claim 6 of the present invention, in the measuring system as defined in Claim 1, an operating lever for switching a language of a voice is arranged so as to be covered up with an exterior case of the complementary device. Therefore, it can be prevented that a user accidentally switches a voice language.

According to Claim 7 of the present invention, in the measuring system as defined in Claim 1, a slope with a tapered shape is provided in the neighborhood of an opening for the measuring device to be inserted, which is provided in the complementary device, in the direction where the measuring device is inserted. Therefore, the slope provided in the complementary device serves as a guide, thereby enabling a reliable integration of the measuring device and the complementary device.

According to Claim 8 of the present invention, in the measuring system as defined in Claim 1, the measuring device and the complementary device have mechanisms for being engaged with each other when being combined. Therefore, it is possible to realize a simple integration and a strong fixation of the measuring device and the complementary device.

According to Claim 9 of the present invention, in the measuring system as defined in Claim 1, an operating part for releasing a combination of the measuring device and the complementary device is provided on the base side of the complementary device. Therefore, it can be prevented that the operating part is operated when unexpected to release a combination of the measuring device and the complementary device.

According to Claim 10 of the present invention, in the measuring system as defined in Claim 1, on the base side of the measuring device, there is provided a concave portion which is locked with a locking means on the complementary device side when being combined with the complementary device. Therefore, a combination of the measuring device and the complementary device can be released by a simple operation.

According to Claim 11 of the present invention, in the measuring system as defined in Claim 1, the measuring device can be inserted into the complementary device only from one direction of the measuring device when the measuring device and the complementary device are combined with each other. Therefore, it can be prevented that the measuring device is wrongly inserted into the complementary device, thereby preventing breakage of both devices.

According to Claim 12 of the present invention, in the measuring system as defined in Claim 1, the measuring device and the complementary device are provided with mechanisms for preventing a wrong insertion of the measuring device into the complementary device. Therefore, it can be prevented that the measuring device is wrongly inserted into the complementary equipment, thereby preventing breakage of both devices.

According to Claim 13 of the present invention, in the measuring system as defined in Claim 1, a rib for regulating the distance to the measuring device is provided on the inner wall of the complementary device. Therefore, it can be prevented that the measuring device is excessively inserted in the complementary device, thereby preventing breakage of both devices.

According to Claim 14 of the present invention, in the measuring system as defined in Claim 1, the measuring device and the complementary device are driven by a battery provided on the complementary device side when the measuring device and the complementary device are combined. Therefore, a battery on the measuring device side is not required when the measuring device and the complementary device are combined.

According to Claim 15 of the present invention, in the measuring system as defined in Claim 14, the measuring device is combined with the complementary device with its cover for a battery storage part and a battery removed. Therefore, the presence or absence of the battery of the measuring device can be detected when the measuring device and the complementary device are combined, whereby it can be prevented that the battery is forgotten to be removed from the measuring device.

According to Claim 16 of the present invention, in the measuring system as defined in Claim 15, the cover for the battery storage part, which cover is provided in the measuring device, is shared as a cover for a battery storage part for storing a battery for the complementary device. Therefore, it can be prevented that the cover for the battery storage part, which cover is removed from the measuring device, is lost when the measuring device and the complementary device are combined.

According to Claim 17 of the present invention, in the measuring system as defined in Claim 15, the cover for the battery storage part of the measuring device is provided with a combining means for combining the cover with the measuring device and a combination releasing means for removing the cover for the battery storage part from the measuring device. Therefore, it can be prevented that the cover for the battery storage part is accidentally removed from the measuring device.

According to Claim 18 of the present invention, in the measuring system as defined in Claim 15, the complementary device is provided with a storage part for the battery removed from the measuring device. Therefore, loss of the battery of the measuring device and an accident due to its scatter can be prevented.

According to Claim 19 of the present invention, in the measuring system as defined in Claim 16, a tip shape of a rib provided on the inner wall of the cover for the battery storage part has a flat part and a circular part for pressing plural kinds of accessories. Therefore, even when batteries of different shapes are used between the measuring device and the complementary device, the batteries of respective shapes can be held reliably.

According to Claim 20 of the present invention, in the measuring system as defined in Claim 1, pattern lengths in a connector part for communication between the measuring device and the complementary device provided in the measuring device are different in a sliding direction of the measuring device when the measuring device is combined with the complementary device. Therefore, an electrode of the connector part can be connected in a desired order.

According to Claim 21 of the present invention, in the measuring system as defined in Claim 1, a terminal on the measuring device side for battery contact between the measuring device and the complementary device is formed at a portion of a main printed board of the measuring device. Therefore, easier assembly can be realized due to reduction in parts count and assembly man-hour of the measuring device.

According to Claim 22 of the present invention, in the measuring system as defined in Claim 1, a terminal on the measuring device side for electrically connecting the measuring device to the complementary device is provided in a battery storage part on the measuring device side. Therefore, it can be prevented that a user improperly touches a terminal part when only the measuring device is used.

According to Claim 23 of the present invention, in the measuring system as defined in Claim 1, when measurement is not started while the measuring device is on standby for measurement, the complementary device promotes measurement start by voice. Therefore, it can be prevented that a measurement operator interrupts the operation halfway through the measurement procedure.

According to Claim 24 of the present invention, in the measuring system as defined in Claim 1, the remaining time is informed by voice by the complementary device during measurement by the measuring device. Therefore, a measurement operator can be easily informed of the elapsed time of the operation.

According to Claim 25 of the present invention, in the measuring system as defined in Claim 1, cutoff of power is informed by voice by the complementary device when measurement by the measuring device is finished. Therefore, a measurement operator can be easily informed that power of the measuring device is cut off.

According to Claim 26 of the present invention, in the measuring system as defined in Claim 1, the initial setting of the measuring device such as clock setting can be performed by an operating part on the complementary device side. Therefore, it is easy for an operator who needs the complementary device to perform time setting of the measuring device.

According to Claim 27 of the present invention, in the measuring system as defined in Claim 1, when the initial setting of the measuring device such as clock setting is not performed when the complementary device and the measuring device are connected, a need of the initial setting is informed by voice by the complementary device. Therefore, it can be avoided that an operator who needs the complementary device improperly performs measurement without performing time setting.

### BRIEF DESCRIPTION OF DRAWINGS

Figure 1 is a perspective view of a measuring device and a complementary device constituting a measuring system according to the present invention.
Figure 2 is a perspective view of the measuring system according to the present invention.
Figure 3 is a perspective view of the measuring device and the complementary device constituting the measuring system according to the present invention, as seen from the base direction.
Figure 4 is a sectional view of the complementary device according to the present invention.
Figure 5 is a sectional view of a battery storage part of the measuring device or the complementary device according to the present invention.
Figure 6 is a perspective view of a conventional measuring device.
Figure 7 is a perspective view of the reverse face of the conventional measuring device.
Figure 8 is an exploded perspective view of a biosensor.
Figure 9 is a perspective view of a conventional measuring device provided with a voice output function.

### BEST MODE TO EXECUTE THE INVENTION

Hereinafter, a measuring system according to the present invention will be specifically described based on figures.

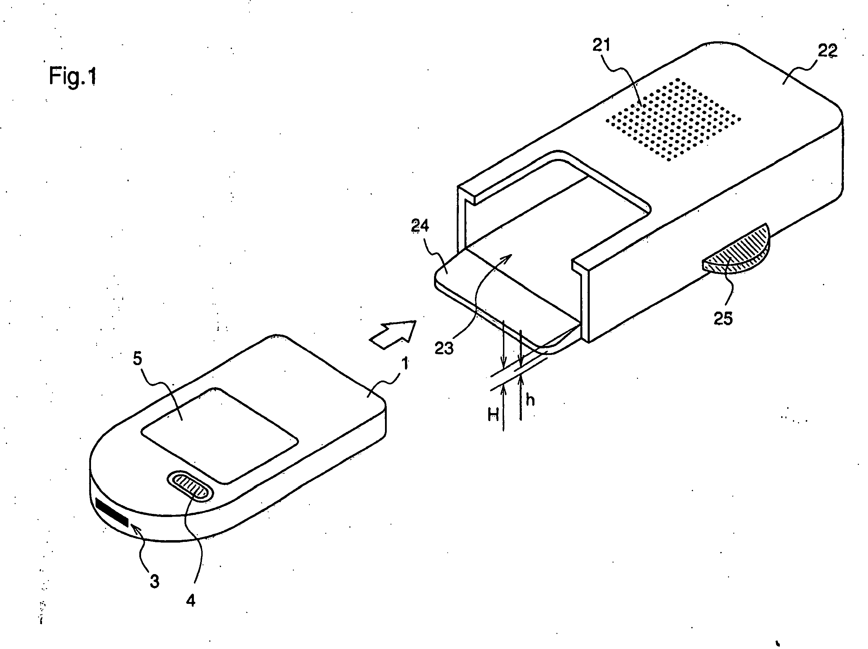

Figures 1 to 3 illustrate the measuring system according to the present invention. In the figures, the same reference numerals as those shown in figures 6 and 7 denote the same or corresponding parts.

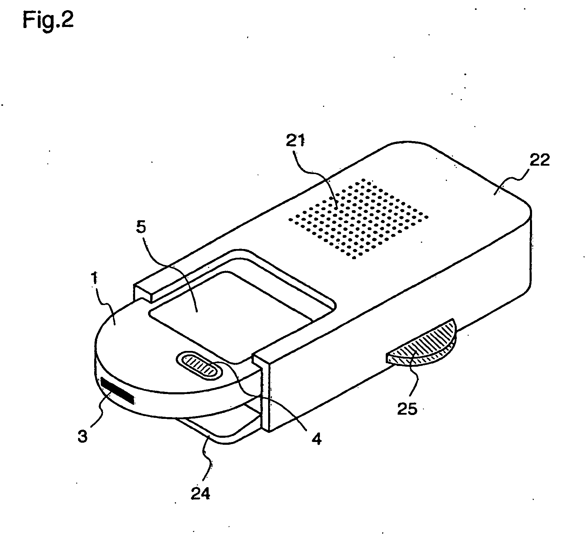

As shown in figure 1, a measuring device 1 is provided with a function of enabling measurement by itself, and a biosensor 2 is inserted in a sensor insertion slot 3 and a prescribed operation is performed, thereby displaying information such as a measurement result on a display means 5. A complementary device 22 can be combined with the measuring device 1, and can receive information such as a measurement result from the measuring device 1 or perform guidance of a measuring operation in a state of being combined with the measuring device 1 as shown in figure 2.

An opening 23 in which the measuring device 1 is inserted is provided in the front of the complementary device 22, and the opening 23 has a slope part 24 which is formed so that the height H at the rear part is higher than the height h at the front part. An electronic circuit which generates a result obtained by communication with a communication part (both of them not shown) of the measuring device 1 or guidance of a measuring operation as a voice is provided inside the complementary device 22, and the output is performed through a voice output part 21 on the top surface of the complementary device 22. The top surface of the complementary device 22 is shaped so that the display means 5 of the measuring device 1 can be seen even after integration, and an operating part 25 is provided on the side of the complementary device 22.

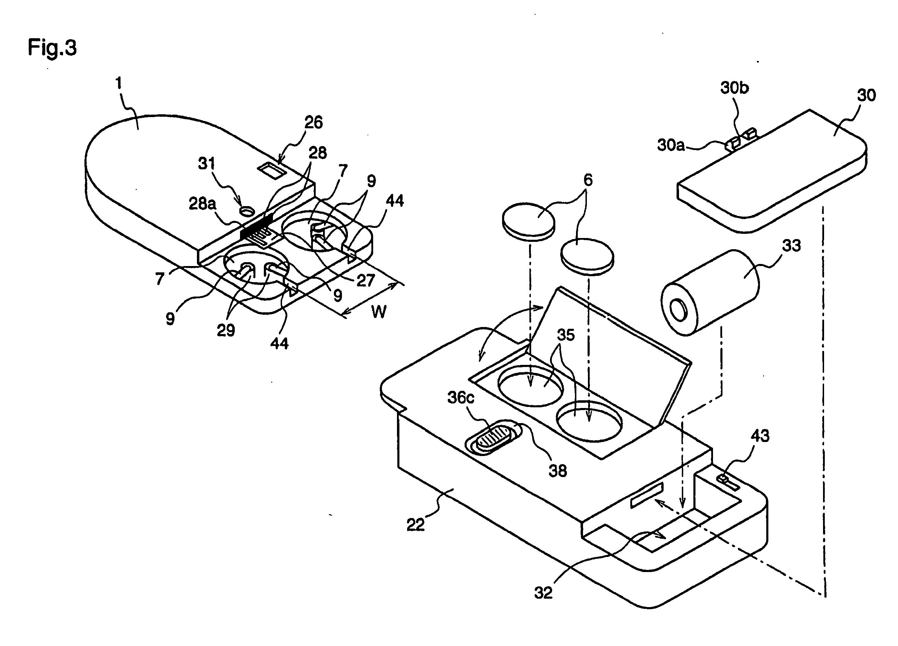

A concave portion 26, a battery storage part 7, and a connector part 27 for making electrical contact with the complementary device 22 are provided on the reverse face of the measuring device 1 as shown in figure 3.

Inside the measuring device 1, there are provided a measuring part comprising a converting means for converting an electrical resistance value of liquid to be examined, which liquid is obtained by the biosensor 2, to a characteristic value of the liquid to be examined, a storage means of the characteristic value, and a calculating means for performing various arithmetical operations such as an average value calculation based on a value stored in the storage means or the characteristic value obtained by measurement, a control means of the display means 5, a main printed board (not shown) provided with an electronic circuit such as a communicating means for communicating with the complementary device 22, and an electrode part (not shown). The connector part 27 is formed at a portion of the main printed board unitedly, and a pattern 28a for a ground connection is constructed to be longer than the other patterns.

A battery 33 is stored in a battery storage part 32 on the reverse face of the complementary device 22. A change-over switch 43 for switching a language of a voice to be outputted is provided at a part which is covered with a back cover 30.

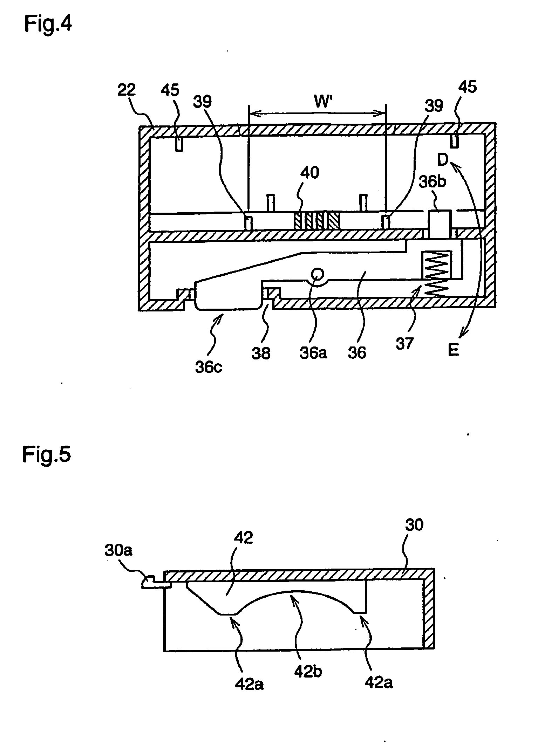

Figure 4 is a sectional view of the complementary device 22 as seen from the opening 23 side. Inside the complementary device 22, a lock lever 36 is provided in a state of being held with a supporting point 36a as a center, and a lock part 36b is usually energized in the direction of D due to an action of a spring 37. A flat part 36c is provided on the opposite side of the lock part 36b with the supporting point 36a therebetween to be located inside the concave portion 38 at the bottom of the complementary device 22. A reception part 40 for receiving the connector part 27 of the measuring device 1 is provided in the inner part of the opening 23. A communicating means (not shown) for communicating with the measuring device 1 is provided inside the complementary device 22, and communication is performed through the connector part 27 and the reception part 40. A distance regulation rib 45 for regulating the distance between the measuring device 1 and the reception part 40 side is provided on the internal top surface of the opening 23.

When the back cover 30 is attached to the measuring device 1, it is fixed to the measuring device 1 due to the action of a lock claw 30a as shown in figure 5. While the battery 6 is pressed against the contact point 9 by a linear part 42a of a rib 42 at this time, contact pressure between the contact point 9 and the battery 6 is appropriately kept by that the projection part 29 provided in the lower part of the contact point 9 makes contact with the contact point 9.

An operation of the measuring system having the above-described construction according to the present invention will be described.

When the measuring device 1 is integrated with the complementary device 22, a flat part 30b of the back cover 30 is pressed from an opening 31 of the measuring device 1 so that a lock is released, the back cover 30 and the battery 6 are removed from the measuring device 1, and the measuring device 1 is inserted from the opening 23 of the complementary device 22. At this time, the measuring device 1 is led into the inside of the complementary device 22 by a slope part 24 of the complementary device 22. When the measuring device 1 is pushed further, a groove part 44 having the groove width W in the measuring device 1 and a guide rib 39 having the width W' equal to the above-mentioned width W in the complementary device 22 are combined, so that the connector part 27 of the measuring device 1 is led to the reception part 40 of the complementary device 22, the pattern 28a first makes contact with an electrode of the reception part 40 to establish a ground connection, and thereafter the rest patterns 28 are connected with the electrode.

After the connection between the connector part 27 and the reception part 40 is completed, a lock part 36b of the lock lever 36 of the complementary device 22 and the concave portion 26 on the base of the measuring device 1 are engaged with each other, thereby fixing the measuring device 1 to the complementary device 22.

In a state where the measuring device 1 is locked in the complementary device 22, the flat part 36C of the lock lever 36 is made not to protrude from the base of the complementary device 22 in the concave portion 38 on the base of the complementary device 22, so that the lock lever 36 is not released improperly.

The back cover 30 removed from the measuring device 1 is attached to the complementary device 22 as a cover for the battery storage part 32 of the complementary device 22, and at this time the battery 33 is held by a curved part 42b of the rib 42 on the back cover 30. The battery 6 removed from the measuring device 1 is stored in a storage part 35 provided in the complementary device 22 at the same time.

When being integrated, the measuring device 1 and the complementary device 22 are driven by the battery 33 on the complementary device 22 side.

In a state where the measuring device 1 and the complementary device 22 are combined, the biosensor 2 is attached in the sensor insertion slot 3 of the measuring device 1 to measure a sample, and at this time voice guidance for a measuring operation or a measurement result is outputted by voice from the voice output part 21. When measurement is not started while the measuring device 1 is on standby for measurement, the complementary device 22 promotes measurement start by voice. The remaining time until a measurement result is outputted is notified by voice from the voice output means 21 of the optional device 22 during measurement, whereby a measurement operator can be easily informed of the elapsed time of the operation, and cutoff of power is informed when measurement is finished, whereby the elapsed time since a start of the measurement or the like is also notified from the voice output part 21 of the complementary device 22.

By operating the operating part 25 of the complementary device 22, various setting operations such as volume setting of a voice to be outputted or setting of output repetition can be performed. Further, by operating the change-over switch 43, a language of a voice to be outputted can be switched.

The initial setting of the measuring device 1 such as clock setting can be performed arbitrarily by rotation and push operations of the operating part 25 on the complementary device 22 side, thereby correctly storing an acquisition date of a measurement value of measurement data or the like, while, when the initial setting of the measuring device 1 such as clock setting is not performed when the complementary device 22 and the measuring device 1 are connected with each other, a need of the initial setting is notified from the voice output means 21, thereby avoiding that an operator who needs the complementary device 22 improperly performs measurement without performing time setting.

When an integration of the complementary device 22 and the measuring device 1 is released, the flat part 36c of the lock lever 36 in the complementary device 22 is pressed, so that the lock lever 36 is rotated in the direction of an arrow E with the supporting point 36a as a center, whereby the lock part 36b and the concave portion 26 of the measuring device 1 are disengaged, and the measuring device 1 can be removed.

As described above, the measuring system according to the first embodiment of the present invention comprises a measuring device 1 which takes a substance to be measured and analyzes components of the substance to be measured, and a complementary device 22 which can generate a voice when combined with the measuring device, and is able to announce a measuring procedure or a measurement result in the measuring device 1 by voice by the complementary device 22, whereby even a weak-sighted person or those unfamiliar with handling of the device can measure accurately and recognize a measurement result.

The measuring device 1 and the complementary device 22 having a voice function can be integrated and separated, whereby, even when plural users require the voice function, only the measuring device 1 which manages measurement data has to be held by each person, and the complementary device 22 can be shared, resulting in reduction of user's burden. Further, when the voice output part 21 has a breakdown, only the complementary device 22 has to be repaired and exchanged, and the measuring device 1 can be used continuously, thereby reducing inconvenience for a user.

The groove part 44 is provided in the measuring device 1, and the guide rib 39 is provided on the complementary device 22 side, whereby, when a conventional measuring device or other models, for example, are to be combined with the complementary device 22, the guide rib 39 serves as a stopper, and thus a wrong insertion of the measuring device 1 can be prevented.

When the back cover 30 is not removed from the measuring device 1 at the integration of the measuring device 1 and the complementary device 22, the guide rib 39 abuts against the back cover 30, thereby preventing an irregular insertion of the measuring device 1. The same effect can be obtained also when the battery 6 is not removed from the measuring device 1, when the direction of the measuring device 1 is irregular, or when a measuring device which is not provided with the groove 44 is to be inserted.

The back cover 30 of the measuring device 1 is removed to be attached to the battery storage part 32 of the complementary device 22 when the measuring device 1 and the complementary device 22 are integrated, thereby preventing loss of the back cover 30. At this time, the battery 6 removed from the measuring device 1 is stored in the battery storage part 35 of the complementary device 22, thereby preventing loss of the battery and an accident due to its scatter.

The flat part 30b of the back cover 30 is pressed from the opening 31 of the measuring device 1 so that a lock is released when the back cover 30 is removed from the measuring device, whereby the back cover is not accidentally removed, resulting in prevention of an accident due to scatter of the battery.

The pattern for a ground connection is constructed to be longer than the other patterns in the connector part 27 of the measuring device 1, whereby, when the measuring device 1 and the complementary device 22 are combined, the ground connection is initially established and connections of the rest of the patterns are completed thereafter, resulting in an electrically stable and reliable connection.

The connector part 27 is formed at a portion of a main substrate of the measuring device 1 unitedly, thereby realizing simplification of assembly processes due to reduction in parts count, assembly man-hour, and the like.

### APPLICABILITY IN INDUSTRY

As described above, according to a measuring system of the present invention, even when plural users require a voice function, only a measuring device which manages measurement data has to be held by each person, and a complementary device can be shared, thereby reducing user's economical burden. Further, when a voice output part has a breakdown, only the complementary device has to be repaired and exchanged, and the measuring device can be used continuously, thereby enhancing convenience for a user.

## Claims

1. A measuring system comprising a measuring device (1) adapted to receive a biosensor (2) for obtaining a liquid to be measured, adapted to analyze components of the liquid to be measured, and adapted to display its measurement result, and a complementary device (22) which is detachably connected to said measuring device (1) and which has a voice output means (21) for generating a voice when combined with the measuring device (1), wherein
the complementary device (22) is adapted to complementarily announce by voice a measuring procedure or a measurement result obtained by the measuring device (1).

2. The measuring system as defined in Claim 1, wherein
the measuring device (1) has a means (27) for establishing communication of information about measurement with the complementary device (22).

3. The measuring system as defined in any one of the preceding claims, wherein
the complementary device (22) has a means (40) for establishing communication of information about measurement with the measuring device (1).

4. The measuring system as defined in any one of the preceding claims, wherein
an operating part (25) for operating the voice output means (21) is arranged on the side of the complementary device (22).

5. The measuring system as defined in any one of the preceding claims, wherein
the complementary device (22) has a function for switching a language of a voice.

6. The measuring system as defined in any one of the preceding claims, wherein
an operating lever (43) for switching a language of a voice is arranged so as to be covered up with an exterior case of the complementary device (22).

7. The measuring system as defined in any one of the preceding claims, wherein
a slope (24) with a tapered shape is provided in the neighborhood of an opening (23) for the measuring device (1) to be inserted, which is provided in the complementary device (22), in the direction where the measuring device (1) is inserted.

8. The measuring system as defined in any one of the preceding claims, wherein
the measuring device (1) and the complementary device (22) have mechanisms (36) for being engaged with each other when being combined.

9. The measuring system as defined in any one of the preceding claims, wherein
an operating part (36c) for releasing a combination of the measuring device (1) and the complementary device (22) is provided on the base side of the complementary device (22).

10. The measuring system as defined in any one of the preceding claims, wherein,
on the base side of the measuring device (1), there is provided a concave portion (26) which is locked with a locking means (36b) on the complementary device side when being combined with the complementary device (22).

11. The measuring system as defined in any one of the preceding claims, wherein
the measuring device (1) can be inserted into the complementary device (22) only from one direction of the measuring device (1) when the measuring device (1) and the complementary device (22) are combined with each other.

12. The measuring system as defined in any one of the preceding claims, wherein
the measuring device (1) and the complementary device (22) are provided with mechanisms (39, 44) for preventing a wrong insertion of the measuring device (1) into the complementary device (22).

13. The measuring system as defined in any one of the preceding claims, wherein
a rib (45) for regulating the distance to the measuring device (1) is provided on the inner wall of the complementary device (22).

14. The measuring system as defined in any one of the preceding claims, wherein
the measuring device (1) and the complementary device (22) are driven by a battery (33) provided on the complementary device side when the measuring device (1) and the complementary device (22) are combined.

15. The measuring system as defined in Claim 14, wherein
the measuring device (1) is combined with the complementary device (22) with its cover (30) for a battery storage part (7) and a battery (6) removed.

16. The measuring system as defined in Claim 15, wherein
the cover (30) for the battery storage part (7), which cover is provided in the measuring device (1), is shared as a cover for a battery storage part (32) for storing a battery (33) of the complementary device (22).

17. The measuring system as defined in Claim 15, wherein
the cover (30) for the battery storage part (7) of the measuring device (1) is provided with a combining means (30a) for combining the cover (30) with the measuring device (1) and a combination releasing means (30b) for removing the cover (30) for the battery storage part (7) from the measuring device (1).

18. The measuring system as defined in Claim 15, wherein
the complementary device (22) is provided with a storage part (35) for the battery (6) removed from the measuring device (1).

19. The measuring system as defined in Claim 16, wherein
a tip shape of a rib (42) provided on the inner wall of the cover (30) of the battery storage part (7, 32) has a flat part (42a) and a circular part (42b) for pressing plural kinds of accessories (6, 33).

20. The measuring system as defined in any one of the preceding claims, wherein
pattern (28, 28a) lengths in a connector part (27) for communication between the measuring device (1) and the complementary device (22) provided in the measuring device (1) are different in a sliding direction of the measuring device (1) when the measuring device (1) is combined with the complementary device (22).

21. The measuring system as defined in any one of the preceding claims, wherein
a terminal on the measuring device side for battery contact between the measuring device (1) and the complementary device (22) is formed at a portion of a main printed board of the measuring device (1).

22. The measuring system as defined in any one of the preceding claims, wherein
a terminal on the measuring device side for electrically connecting the measuring device (1) to the complementary device (22) is provided in a battery storage part on the measuring device side.

23. The measuring system as defined in any one of the preceding claims, wherein,
when measurement is not started while the measuring device (1) is on standby for measurement, the complementary device (22) promotes measurement start by voice.

24. The measuring system as defined in any one of the preceding claims, wherein
the remaining time is informed by voice by the complementary device (22) during measurement by the measuring device (1).

25. The measuring system as defined in any one of the preceding claims, wherein
cutoff of power is informed by voice by the complementary device (22) when measurement by the measuring device (1) is finished.

26. The measuring system as defined in any one of the preceding claims, wherein
the initial setting of the measuring device (1) such as clock setting can be performed by an operating part on the complementary device side.

27. The measuring system as defined in any one of the preceding claims, wherein,
when the initial setting of the measuring device (1) such as clock setting is not performed when the complementary device (22) and the measuring device (1) are connected, a need of the initial setting is informed by voice by the complementary device (22).

## Patentansprüche

1. Messsystem, aufweisend eine Messvorrichtung (1), angepasst zur Aufnahme eines Biosensors (2) zum Erhalten einer zu messenden Flüssigkeit, angepasst zum Analysieren von Bestandteilen der zu messenden Flüssigkeit, und angepasst zum Anzeigen seines Messergebnisses, und eine ergänzende Vorrichtung (22), die abnehmbar mit der Messvorrichtung (1) verbunden ist und die bei Kombination mit der Messvorrichtung (1) ein Sprachausgabemittel (21) zum Erzeugen einer Stimme hat, wobei
die ergänzende Vorrichtung (22) angepasst ist für ein ergänzendes Bekanntgeben durch Sprachausgabe eines Messvorgangs oder eines von der Messvorrichtung (1) erhaltenen Messergebnisses.

2. Messsystem nach Anspruch 1, wobei
die Messvorrichtung (1) Mittel (27) zur Erstellung einer Kommunikation mit Information über Messung mit der ergänzenden Vorrichtung (22) aufweist.

3. Messsystem nach einem der vorhergehenden Ansprüche, wobei
die ergänzende Vorrichtung (22) Mittel (40) zur Erstellung einer Kommunikation mit Information über Messung mit der Messvorrichtung (1) aufweist.

4. Messsystem nach einem der vorhergehenden Ansprüche, wobei
ein Bedienteil (25) zum Betreiben des Sprachausgabemittels (21) an der Seite der ergänzenden Vorrichtung (22) angeordnet ist.

5. Messsystem nach einem der vorhergehenden Ansprüche, wobei
die ergänzende Vorrichtung (22) eine Funktion zum Schalten einer Sprache einer Stimme aufweist.

6. Messsystem nach einem der vorhergehenden Ansprüche, wobei
ein Bedienungshebel (43) zum Schalten einer Sprache so angeordnet ist, dass von einem äußeren Gehäuse der ergänzenden Vorrichtung (22) verdeckt ist.

7. Messsystem nach einem der vorhergehenden Ansprüche, wobei
eine Neigung (24) mit kegeliger Form in der Nähe einer Öffnung (23) für ein Einsetzen der Messvorrichtung (1) bereitgestellt ist, die in der ergänzenden Vorrichtung (22) in der Richtung, in der die Messvorrichtung (1) eingesetzt ist, bereitgestellt ist.

8. Messsystem nach einem der vorhergehenden Ansprüche, wobei
die Messvorrichtung (1) und die ergänzende Vorrichtung (22) Mechanismen für ein Eingreifen miteinander bei einer Kombination aufweisen.

9. Messsystem nach einem der vorhergehenden Ansprüche, wobei
ein Bedienteil (36c) zum Freigeben einer Kombination der Messvorrichtung (1) und der ergänzenden Vorrichtung (22) auf der Unterseite der ergänzenden Vorrichtung (22) bereitgestellt ist.

10. Messsystem nach einem der vorhergehenden Ansprüche, wobei
auf der Unterseite der Messvorrichtung (1) ein konkaver Teil (26) bereitgestellt ist, der mit einem Sperrmittel (36b) auf der Seite der ergänzenden Vorrichtung gesperrt ist, wenn mit der ergänzenden Vorrichtung (22) kombiniert.

11. Messsystem nach einem der vorhergehenden Ansprüche, wobei
die Messvorrichtung (1) nur von einer Richtung der Messvorrichtung (1) aus in die ergänzende Vorrichtung (22) eingesetzt werden kann, wenn die Messvorrichtung (1) und die ergänzende Vorrichtung (22) miteinander kombiniert werden.

12. Messsystem nach einem der vorhergehenden Ansprüche, wobei
die Messvorrichtung (1) und die ergänzende Vorrichtung (22) mit Mechanismen (39, 44) zum Verhindern eines fehlerhaften Einsetzens der Messvorrichtung (1) in die ergänzende Vorrichtung (22) bereitgestellt sind.

13. Messsystem nach einem der vorhergehenden Ansprüche, wobei
eine Rippe (45) zum Regeln des Abstands zur Messvorrichtung (1) auf der inneren Wand der ergänzenden Vorrichtung (22) bereitgestellt it.

14. Messsystem nach einem der vorhergehenden Ansprüche, wobei
die Messvorrichtung (1) und die ergänzende Vorrichtung (22) von einer Batterie (33) angetrieben werden, die an der Seite der ergänzenden Vorrichtung angeordnet ist, wenn die Messvorrichtung (1) und die ergänzende Vorrichtung (22) kombiniert sind.

15. Messsystem nach Anspruch 14, wobei
die Messvorrichtung (1) mit der ergänzenden Vorrichtung (22) mit deren Deckel (30) für einen Batterie-Aufbewahrungsteil (7) und einer entfernten Batterie (6) kombiniert ist.

16. Messsystem nach Anspruch 15, wobei
der Deckel (30) für den Batterie-Aufbewahrungsteil (7), der in der Messvorrichtung (1) bereitgestellt ist, gemeinsam als ein Deckel für einen Batterie-Aufbewahrungsteil (32) zum Aufbewahren einer Batterie (33) auf der ergänzenden Vorrichtung (22) ist.

17. Messsystem nach Anspruch 15, wobei
der Deckel (30) für den Batterie-Aufbewahrungsteil (7) der Messvorrichtung (1) mit einem Kombinationsmittel (30a) zum Kombinieren des Deckels (30) mit der Messvorrichtung (1) und einem Kombinations-Freigabe-Mittel (30b) zum Entfernen des Deckels (30) für den Batterie-Aufbewahrungsteil (7) der Messvorrichtung (1) bereitgestellt ist.

18. Messsystem nach Anspruch 15, wobei
die ergänzende Vorrichtung (22) mit einem Aufbewahrungsteil (35) für die von der Messvorrichtung (1) entfernte Batterie (6) bereitgestellt ist.

19. Messsystem nach Anspruch 16, wobei
eine Spitzenform einer Rippe (42) die auf der inneren Wand des Deckels (30) des Batterie-Aufbewahrungsteils (7, 32) bereitgestellt ist, einen abgeflachten Teil (42a) und einen runden Teil (42b) zum Pressen mehrerer Sorten von Zubehören (6, 33) aufweist.

20. Messsystem nach einem der vorhergehenden Ansprüche, wobei
in der Messvorrichtung (1) bereitgestellte Musterlängen (28, 28a) in einem Anschlussteil (27) für Kommunikation zwischen der Messvorrichtung (1) und der ergänzenden Vorrichtung (22) unterschiedlich in einer Gleitrichtung der Messvorrichtung (1) sind, wenn die Messvorrichtung (1) mit der ergänzenden Vorrichtung (22) kombiniert ist.

21. Messsystem nach einem der vorhergehenden Ansprüche, wobei
ein Anschlussteil auf der Seite der Messvorrichtung für Batteriekontakt zwischen der Messvorrichtung (1) und der ergänzenden Vorrichtung (22) an einem Teil einer gedruckten Hauptleiterplatte der Messvorrichtung (1) gebildet ist.

22. Messsystem nach einem der vorhergehenden Ansprüche, wobei
ein Anschlussteil auf der Seite der Messvorrichtung für elektrischen Anschluss der Messvorrichtung (1) an der ergänzenden Vorrichtung (22) in einem Batterie-Aufbewahrungsteil auf der Seite der Messvorrichtung bereitgestellt ist.

23. Messsystem nach einem der vorhergehenden Ansprüche, wobei,
wenn eine Messung nicht gestartet ist, während sich die Messvorrichtung (1) in Bereitschaft für Messung befindet, fördert die ergänzende Vorrichtung (22) einen Messstart durch Sprache.

24. Messsystem nach einem der vorhergehenden Ansprüche, wobei
die verbleibende Zeit durch Sprache von der ergänzenden Vorrichtung (22) während der Messung durch die Messvorrichtung (1) mitgeteilt wird.

25. Messsystem nach einem der vorhergehenden Ansprüche, wobei
das Abschalten von Strom durch Sprache von der ergänzenden Vorrichtung (22) mitgeteilt wird, wenn die Messung durch die Messvorrichtung (1) beendet ist.

26. Messsystem nach einem der vorhergehenden Ansprüche, wobei
die Ausgangseinstellung der Messvorrichtung (1), wie Einstellen der Uhr, durch ein Bedienteil auf der Seite der ergänzenden Vorrichtung ausgeführt werden kann.

27. Messsystem nach einem der vorhergehenden Ansprüche, wobei,
wenn die Ausgangseinstellung der Messvorrichtung (1), wie Einstellen der Uhr, nicht ausgeführt wird, wenn die ergänzende Vorrichtung (22) und die Messvorrichtung (1) miteinander verbunden sind, wird der Bedarf an einer Ausgangseinstellung durch Sprache von der ergänzenden Vorrichtung (22) mitgeteilt.

## Revendications

1. Système de mesure comprenant un dispositif de mesure (1) adapté pour recevoir un biocapteur (2) destiné à obtenir un liquide à mesurer, adapté pour analyser des composants du liquide à mesurer et adapté pour afficher son résultat de mesure, et
un dispositif complémentaire (22) qui est relié de manière amovible audit dispositif de mesure (1) et qui a un moyen de sortie de voix (21) pour générer une voix lorsqu'il est combiné au dispositif de mesure (1), dans lequel
le dispositif complémentaire (22) est adapté pour annoncer de manière complémentaire par la voix une procédure de mesure ou un résultat de mesure obtenu par le dispositif de mesure (1).

2. Système de mesure tel que défini dans la revendication 1, dans lequel
le dispositif de mesure (1) a un moyen (27) pour établir une communication d'informations concernant une mesure avec le dispositif complémentaire (22).

3. Système de mesure tel que défini dans l'une quelconque des revendications précédentes, dans lequel
le dispositif complémentaire (22) a un moyen (40) pour établir une communication d'informations concernant une mesure avec le dispositif de mesure (1).

4. Système de mesure tel que défini dans l'une quelconque des revendications précédentes, dans lequel
une partie d'actionnement (25) pour actionner le moyen de sortie vocale (21) est agencée sur le côté du dispositif complémentaire (22).

5. Système de mesure tel que défini dans l'une quelconque des revendications précédentes, dans lequel
le dispositif complémentaire (22) a une fonction de commutation d'une langue d'une voix.

6. Système de mesure tel que défini dans l'une quelconque des revendications précédentes, dans lequel
un levier d'actionnement (43) pour faire commuter un langage d'une voix est agencé de manière à être recouvert d'un boîtier externe du dispositif complémentaire (22).

7. Système de mesure tel que défini dans l'une quelconque des revendications précédentes, dans lequel
une pente (24) avec une forme conique est prévue au voisinage d'une ouverture (23) pour le dispositif de mesure (1) à insérer, qui est prévue dans le dispositif complémentaire (22), dans la direction où le dispositif de mesure (1) est inséré.

8. Système de mesure tel que défini dans l'une quelconque des revendications précédentes, dans lequel
le dispositif de mesure (1) et le dispositif complémentaire (22) ont des mécanismes (36) pour s'engager l'un avec l'autre lorsqu'ils sont combinés.

9. Système de mesure tel que défini dans l'une quelconque des revendications précédentes, dans lequel
une partie d'actionnement (36c) pour libérer une combinaison du dispositif de mesure (1) et du dispositif complémentaire (22) est prévue sur le côté de base du dispositif complémentaire (22).

10. Système de mesure tel que défini dans l'une quelconque des revendications précédentes, dans lequel,
une partie concave (26), qui est verrouillée avec un moyen de verrouillage (36b) sur le côté de dispositif complémentaire lorsque le dispositif de mesure est combiné avec le dispositif complémentaire (22), est prévue sur le côté de base du dispositif de mesure (1).

11. Système de mesure tel que défini dans l'une quelconque des revendications précédentes, dans lequel
le dispositif de mesure (1) peut être inséré dans le dispositif complémentaire (22) uniquement à partir d'une direction du dispositif de mesure (1) lorsque le dispositif de mesure (1) et le dispositif complémentaire (22) sont combinés l'un avec l'autre.

12. Système de mesure tel que défini dans l'une quelconque des revendications précédentes, dans lequel
le dispositif de mesure (1) et le dispositif complémentaire (22) sont dotés de mécanismes (39, 44) pour empêcher une mauvaise insertion du dispositif de mesure (1) dans le dispositif complémentaire (22).

13. Système de mesure tel que défini dans l'une quelconque des revendications précédentes, dans lequel
une nervure (45) pour réguler la distance par rapport au dispositif de mesure (1) est prévue sur la paroi interne du dispositif complémentaire (22).

14. Système de mesure tel que défini dans l'une quelconque des revendications précédentes, dans lequel
le dispositif de mesure (1) et le dispositif complémentaire (22) sont entraînés par une batterie (33) prévue sur le côté de dispositif complémentaire lorsque le dispositif de mesure (1) et le dispositif complémentaire (22) sont combinés.

15. Système de mesure tel que défini dans la revendication 14, dans lequel
le dispositif de mesure (1) est combiné avec le dispositif complémentaire (22) avec son couvercle (30) pour une partie de stockage de batterie (7) et une batterie (6) retiré.

16. Système de mesure tel que défini dans la revendication 15, dans lequel
le couvercle (30) pour la partie de stockage de batterie (7), lequel couvercle est prévu dans le dispositif de mesure (1), est partagé en tant que couvercle pour une partie de stockage de batterie (32) pour stocker une batterie (33) du dispositif complémentaire (22).

17. Système de mesure tel que défini dans la revendication 15, dans lequel
le couvercle (30) pour la partie de stockage de batterie (7) du dispositif de mesure (1) est doté d'un moyen de combinaison (30a) pour combiner le couvercle (30) avec le dispositif de mesure (1) et un moyen de libération de combinaison (30b) pour retirer le couvercle (30) pour la partie de stockage de batterie (7) du dispositif de mesure (1).

18. Système de mesure tel que défini dans la revendication 15, dans lequel
le dispositif complémentaire (22) est doté d'une partie de stockage (35) pour la batterie (6) retirée du dispositif de mesure (1).

19. Système de mesure tel que défini dans la revendication 16, dans lequel
une forme de pointe d'une nervure (42) prévue sur la paroi interne du couvercle (30) de la partie de stockage de batterie (7, 32) a une partie plate (42a) et une partie circulaire (42b) pour presser plusieurs types d'accessoires (6, 33).

20. Système de mesure tel que défini dans l'une quelconque des revendications précédentes, dans lequel
des longueurs de motif (28, 28a) dans une partie de liaison (27) pour une communication entre le dispositif de mesure (1) et le dispositif complémentaire (22) prévue dans le dispositif de mesure (1) sont différentes dans une direction de coulissement du dispositif de mesure (1) lorsque le dispositif de mesure (1) est combiné avec le dispositif complémentaire (22).

21. Système de mesure tel que défini dans l'une quelconque des revendications précédentes, dans lequel
une borne sur le côté de dispositif de mesure pour un contact de batterie entre le dispositif de mesure (1) et le dispositif complémentaire (22) est formée au niveau d'une partie d'une carte de circuit imprimé principale du dispositif de mesure (1).

22. Système de mesure tel que défini dans l'une quelconque des revendications précédentes, dans lequel
une borne sur le côté de dispositif de mesure pour relier électriquement le dispositif de mesure (1) au dispositif complémentaire (22) est prévue dans une partie de stockage de batterie sur le côté de dispositif de mesure.

23. Système de mesure tel que défini dans l'une quelconque des revendications précédentes, dans lequel,
lorsque la mesure n'a pas commencé pendant que le dispositif de mesure (1) est en attente pour la mesure, le dispositif complémentaire (22) favorise le commencement de la mesure par une voix.

24. Système de mesure tel que défini dans l'une quelconque des revendications précédentes, dans lequel
le temps restant est notifié par une voix par le dispositif complémentaire (22) au cours d'une mesure par le dispositif de mesure (1).

25. Système de mesure tel que défini dans l'une quelconque des revendications précédentes, dans lequel
une coupure de puissance est notifiée par une voix par le dispositif complémentaire (22) lorsque la mesure par le dispositif de mesure (1) est terminée.

26. Système de mesure tel que défini dans l'une quelconque des revendications précédentes, dans lequel
le réglage initial du dispositif de mesure (1) tel qu'un réglage d'horloge peut être effectué par une partie d'actionnement sur le côté de dispositif complémentaire.

27. Système de mesure tel que défini dans l'une quelconque des revendications précédentes, dans lequel,
lorsque le réglage initial du dispositif de mesure (1) tel qu'un réglage d'horloge n'est pas effectué lorsque le dispositif complémentaire (22) et le dispositif de mesure (1) sont reliés, un besoin du réglage initial est notifié par une voix par le dispositif complémentaire (22).
